# EUROPEAN PATENT SPECIFICATION

(11) **EP 2 368 444 B1**
(45) Date of publication and mention of the grant of the patent: **23.12.2015**
(21) Application number: 09834047.4
(22) Date of filing: 27.09.2009
(51) Int. Cl.: A23L 1/28, A23L 1/228, C12N 1/16, C12R 1/865

(54) **YEAST EXTRACT WITH HIGH GLUTAMIC ACID CONTENT AND PRODUCING METHOD THEREOF**
HEFEEXTRAKT MIT HOHEM GLUTAMINSÄUREANTEIL UND VERFAHREN ZU SEINER HERSTELLUNG
EXTRAIT DE LEVURE À TENEUR ÉLEVÉE EN ACIDE GLUTAMIQUE ET SON PROCÉDÉ DE PRODUCTION

(30) Priority: 24.12.2008 CN 200810189433
(43) Date of publication of application: 28.09.2011
(73) Proprietor: Angelyeast Co., Ltd., Yichang Hubei 443003 (CN)
(72) Inventor: YU, Xuefeng, Yichang Hubei 443003 (CN); LI, Zhihong, Yichang Hubei 443003 (CN); YU, Minghua, Yichang Hubei 443003 (CN); YAO, Juan, Yichang Hubei 443003 (CN); LI, Ku, Yichang Hubei 443003 (CN); LI, Pei, Yichang Hubei 443003 (CN); TANG, Guanqun, Yichang Hubei 443003 (CN)
(74) Representative: Long, Giorgio
(86) International application number: PCT/CN2009/074263
(87) International publication number: WO 2010/072094

(56) References cited:
- EP-A1- 0 578 572
- WO-A1-2007/073945
- CN-A- 1 977 039
- CN-A- 101 024 818
- CN-A- 101 225 363
- JP-A- 2002 171 961
- JP-A- 2006 129 835
- US-A1- 2002 081 357
- amano enzyme: "yeast extract", , 1 January 2007 (2007-01-01), pages 1-1, XP002673908, Retrieved from the Internet: URL:www.amano-enzyme.co.jp
- T.V.MILIC ET AL.: "utilization of baker's yeast for the production of yeast extract:effects of different enzymatic treatments on solid, protein and carbohydate recovery", J.SERB.CHEM.SOC., vol. 72, no. 5, 1 January 2007 (2007-01-01), pages 451-457, XP002673909,
- DONG, JIAWU ET AL.: 'Research on production ofYeast extract and the use thereof in condiments industry' SCIENCE AND TECHNOLOGY OF FOOD INDUSTRY vol. 24, no. 05, 31 December 2003, pages 103 - 105, XP008141214
- YAN, ZHIYUN ET AL.: 'Natural Flavouring Agents-Yeast Extract' FOOD AND FERMENTATION INDUSTRIES vol. 24, no. 06, 30 June 1998, pages 42 - 48, XP008140898
- HEE JEONG CHAE ET AL.: 'Utilization of brewer's yeast cells for the production of food-grade yeast extract. Part 1. effects of different enzymatic treatments on solid and protein recovery and flavor characteristics' BIORESOURCE TECHNOLOGY vol. 76, no. 3, 28 February 2001, pages 253 - 258, XP009049996
- YOSHIFUMI TAKATSUME ET AL.: 'Enrichment of yeast thioredoxin by green tea extract through activation of Yap I transcription factor in Saccharomyces cerevisiae' JOURNAL OF AGRICULTURAL AND FOOD CHEMISTRY vol. 53, no. 2, 26 January 2005, pages 332 - 337, XP008140802

## Description

### Technical field

The present invention relates to yeast extract and producing method thereof; and more specifically, relates to yeast extract with high glutamic acid content and producing method thereof.

### Background

As a naturally existing amino acid, glutamic acid mainly exists in protein-abundant foods such as mushrooms, kelps, tomato, nuts, beans, meats and most of milk products. In some foods, glutamic acid exists in free form, which is the only form of glutamate that can enhance the fresh flavor of foods. The seasoning effect from tomatoes, fermented bean products, yeast extracts, certain kinds of cheese, and fermented or hydrolyzed protein products (such as soy sauce or bean sauce) owes partly to the existence of glutamic acid.

In recent years, with the improvement of people's living standard, new requirements for the content of diet have been raised. Various foods are developed to becoming more and more natural, convenient, delicious and multi-functional. And food condiments have been changed from the previously used brewing or chemical ones into natural seasoning complexes with advanced taste. Meanwhile, condiments in the market are being developed into advanced seasoning complexes that are delicious, convenient, natural and nutrient. Yeast extract contains amino acids and polypeptides as well as other effective components, like nucleotides, vitamins, organic acids and minerals. With strong and delicious flavor and taste of meat, the yeast extract is an advanced food condiment that has multi-function of nutrient, seasoning and health-care, thus becoming more and more popular among customers.

The use of yeast extract is significantly expanded with the development of biotechnology. The newest generation of yeast extract contains abundant of glutamic acid and nucleotide, further makes its flavor improved. Together with amino acids and polypeptides that exist originally, it can significantly reduce the content of sodium salts while not losing the original flavor. More importantly, with purely natural characteristic, it prevents anxieties for artificial synthesis or chemical additives.

However, the content of sodium glutamate in present yeast extract is generally 4%. More and more food enterprises are demanding for yeast extract with higher sodium glutamate content, thus enhancing its flavor.

Prior-art document JP 2002 171961 discloses a method for producing a yeast extract, which is obtained by subjecting to digestion a variant strain of yeast, which contains at least 15 mg of glutamic acid per gram of dried yeast cell in the cell.

In JP 2006 129835) it is disclosed a process implying the extraction of yeast from food with the use of an acidic aqueous solution, then subjecting the product to centrifugation, bringing the obtained supernatant into contact with basidiomycete-producing enzymes and treating the product with activated carbon. The used yeast is *Candida utilis* instead of *S. cerevisiae.*

The disclosure of Amano enzyme "Yeast extract" retrieved from the Internet (www.amano-enzyme.co.jp) reports the preparation of a yeast extract, mainly focusing on the enhancement of the flavor.

The disclosed process comprises autolysis of a yeast preparation obtained with the treatment of endogenous or exogenous enzymes.

EP 0 578 572 discloses a process for the hydrolysis of a starting proteic raw material in an aqueous medium, a sequence of hydrolysis steps using proteases and peptidases, wherein baker yeast is then added to the obtained preparation, then an agent capable of producing a plasmolysant autolysis, particularly ethyl acetate, is added so that glutaminase is released; after that, glutaminase is left reacting so that glutamine is converted to glutamic acid.

The publication of T.V. Milic et al (J. Serb. Chem. Soc., 2007) discloses the preparation of an yeast extract using two enzymes: papain and lyticase, wherein the optimal concentration of papain is 2.5%.

### Disclosure of the present invention

To solve the problem of low glutamic acid content in the present yeast extract, the present invention aims to provide a yeast extract with high glutamic acid content and producing method thereof.

One object of the present invention is to provide yeast extract that is produced from yeast strain, which belongs to *Saccharomyces cerevisiae* (Z2.4) under CCTCC NO: M205130. The yeast strain is cultivated in culture media rich in nitrogen sources and then produces yeast culture with protein content of 60% or more, which serves as raw material for the production of yeast extract. The yeast extract provided according to the present invention possesses glutamic acid content of more than 10%.

Another object of the present invention is to provide a producing method for yeast extract, wherein said yeast extract is produced with a yeast strain culture as raw material, wherein the deposit accession number of said yeast strain is CCTCC NO: M205130 and the protein content of said culture is higher than 60%, said method comprising: A) cultivating yeast strain whose deposit accession number is CCTCC NO: M205130 in culture media containing nitrogen sources, obtaining culture and preparing said culture into yeast cream, wherein aqua ammonia as the nitrogen source in culture media accounts for 2% or more of the total mass of culture media; B) adjusting the pH value of yeast cream to pH4.5-6.8 and heating it to 45°C-60°C; C) adding protease and glutamine transaminase to perform autolytic enzymolysis, wherein said protease is papain and is added in an amount of 1.5‰ relative to the total weight of yeast in said yeast cream; D) raising the temperature to 60°C and performing centrifugation; and E) obtaining said yeast extract after sterilization, concentration and spray drying.

According to an embodiment of the present invention, the mass ratio of protease and glutamine transaminase added is 1: 1.

According to an embodiment of the present invention, the mass of protease and glutamine transaminase added are both 1.5‰ of the total yeast weight in said yeast cream.

The process employed in the present invention is as follows:

Yeast cream (12-15% of concentration)
Yeast heat at 45-60°C
Enzymolysis with protease and glutamine transaminase
(Autolytic enzymolysis at pH 4.5-6.8 and 45-68°C  for 16-20 hours)
Enzyme deactivation with heat treatment, centrifugation, sterilization,
concentration and spray drying

According to an embodiment of the present invention, autolytic enzymolysis reaction is carried out for about 16-20 hours because in practical production, complete enzymolysis reaction can not be achieved with less than 16 hours; however, it will cause cost increase if the time is over 20 hours.

According to an embodiment of the present invention, the pH value of autolytic enzymolysis reaction is in the range of 4.5-6.8.

Yeast extract produced according to the process of the present invention is abundant of glutamic acid, with content of over 10%, and possesses more delicious flavor and improved capability for food flavor enhancement. The method provided according to the present invention is easy to carry out and can be used for industrialized production of yeast extract with high sodium glutamate content, thus satisfying the forthcoming commercialized production of yeast extract on a large scale.

### Deposit information of yeast in the present invention

Yeast belonging to *Saccharomyces cerevisiae* with latin name of *Saccharomyces cerevisiae*_Hansen Z 2.4 has been deposited in China Center for Type Culture Collection (CCTCC) under accession number CCTCC NO: M205130 on October 25, 2005.

### Embodiments

### I. Properties of the strain

### 1. Mycology characteristics

**Table 1-1 Mycology properties of Saccharomyces cerevisiae CCTCC M205130 of the present invention (I)**

| | |
|---|---|
| Trophocyte shape | Oval or ellipse cells |
| Proliferation style | Sprouting |
| Growth condition | Growing well on liquid or solid culture media |
| Ascospore | 1-4 spherical ascospores with smooth surface |
| Optimum growth conditions | pH5.2, temperature: 28-30°C, 12 Brix wort agar medium |

**Table 1-2 Mycology properties of Saccharomyces cerevisiae CCTCC M205130 of the present invention (II)**

| Items | | Assimilation | Fermentability |
|---|---|---|---|
| Nitrogen sources | Nitrates | - | |
| Carbon sources | Glucose | + | + |
| | Galactose | + | + |
| | Sucrose | + | + |
| | Maltose | + | + |
| | Lactose | - | - |
| | Trehalose | - | - |

With mycology properties mentioned above, the *Saccharomyces cerevisiae* strain CCTCC NO. M205130 according to the present invention is compared to "The yeasts, A Taxonomic Study" (1998, Holland, Lodder J edited, the 4th edition) and identified as belonging to *Saccharomyces cerevisiae* genus.

### 2. Cultivation of the yeast strain

Culture media: 12 Brix of wort agar medium, 2% agar. Adjust pH to 5.2, sterilize with steam at 121°C for 20 min, and then cultivate the above mentioned yeast strain under 28-30°C on routine conditions.

To increase protein content of yeasts, lots of nitrogen sources, namely aqua ammonia, are added into culture media to make sure that nitrogen source in culture media accounts for 2% or more of the total mass of culture media.

Conservation conditions of the strain: conserved in 20% defatted milk with lyophilization method (Vacuum freeze-drying).

The result culture is assayed, by Kjeldah method in a Kjeldahl apparatus, to possess 60% or more of protein content.

Fermenting liquid obtained from yeast fermentation is centrifuged and yeast cream is obtained by gathering centrifugation precipitations (or the heavy phase).

### II. Production of yeast extract

Producing method of yeast extract according to the present invention comprises steps as follows:
A) Cultivating yeast strain with accession number of CCTCC NO: M205130 in culture media containing aqua ammonia, centrifuging the fermenting liquid obtained from above, and obtaining yeast cream by gathering centrifugation precipitations (or the heavy phase):
B) Adjusting the pH value of yeast cream to acid level (pH 4.5-6.8) and heating it to 45°C-60°C to sterilize yeasts; Controlling temperature between 45-60°C, because temperature less than 45°C facilitates the growth of spoilage organisms like gas-producing bacteria, while temperature over 60°C leads to severe protein denaturation and makes degradations difficult;
C) Adding protease and glutamine transaminase into the above mentioned yeast cream to perform autolytic enzymolysis reaction under pH 4.5-6.8, wherein protease is added for the degradation of macromolecule proteins into micromolecule peptides or amino acids, and glutamine transaminase is added to increase free glutamic acid content, with preferred addition ratio of the two as 1:1, preferred autolytic enzymolysis reaction time of 16-20 hours, allowable temperature between 45°C-68°C and preferably between 55°C-65°C, and preferred pH of 6.0-6.5;
D) Deactivating enzymes with heat treatment and centrifuging;
E) Concentrating and spray drying the supernatant solution resulted from centrifugation to obtain yeast extract rich in glutamic acid.

### III. Assays for glutamic acid content

Apparatus: 2700 biochemistry analyzer (YSI corp., USA.)

Reagent: sodium glutamate

Mechanism of the assay: Glutamic acid content in solution is directly fetched with enzyme sensor, wherein glutamic acid oxidase is immobilized on YSI glutamic acid membrane.

Fundamental reaction formula is as follows:

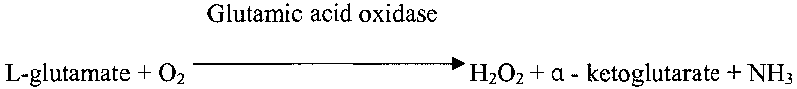

Experimental reagents and apparatus: YSI2357 buffer solution (one bag of YSI2357 in 500 ml high-purified water); L-glutamic acid standard preparation (5mmol/L); YSI2754 glutamic acid assay membrane; YSI biochemistry analyzer.

Experimental parameters: YSI membrane: 2754; measurement range: 0-10 mmol/L; standard solution concentration: 5.0 mmol/L; maximum detection limit: 10.0 mmol/L; precision: 2%; errors between 0-5.0 mmol/L: ±2%; errors between 5.0 mmol/L-10 mmol/L: ±5; useful life of membrane after activation: 7 days; color of O-type membrane: yellow.

Glutamic acid assay method:
1. Prepare 5.0 mmol/L of glutamic acid standard solution and YSI2357 buffer solution with high-purified water and separately load them into specific bottles supplied by YSI biochemistry analyzer.
2. Membrane fixing: Unload the probe of YSI biochemistry analyzer and drop at the center with one drop of NaCl solution specific for YSI biochemistry analyzer. Then open carefully the membrane box and aiming at the probe center, put membrane into probe. After that, drop to the membrane center with one drop of NaCl solution, fix the probe and screw tightly. Press *RUN* button; rinse the membrane for 3 times with solution buffer first and with standard solution for another 3 times.
3. Program and parameter settings for L-glutamic acid assay: Refer to the corresponding program and parameter settings in L-glutamic acid assay chapter in YSI biochemistry analyzer operating manual.
4. Activation of the membrane: Approximately 24 h is needed. The completion of activation is determined if electric current value is usually less than 6. The less is electric current value, the better the activation effect is. Sample assay program can be started to detect samples after activation.
5. Sample assay: Dissolve yeast extract in high purified water with glutamic acid content of 0-5.0 mmol/l. Pour the prepared yeast extract solution into sample well (1# work bench) or the beaker (2# work bench), press *sample* button on the screen and the probe starts to sample and assay. After 30 s, assay results as well as terminal signals will be shown on the screen of the apparatus. Press *sample* button then to start new sample assay.

Examples of the present invention are showed below to further illustrate the present invention in detail. Those examples are provided only for illustration, while not limiting the protection scope of the present invention.

### Example 1

Being used as raw material, yeast cream from *Saccharomyces cerevisiae* strain CCTCC NO. M205130 fermented in molasses with enhanced nitrogen source was diluted to concentration of 13%-15% solids content and maintained for 10 hours under temperature of 48°C -52°C and pH of 5.5, with hydrochloric or sulphuric acid added for pH adjustment. The temperature was then increased to 55°C. Papain was added with content of 1.5‰ by mass of the yeast, and glutamine transaminase added with content of 1.5‰ by mass of the yeast. PH was adjusted to 6.0-6.5 and reaction was carried out for 4 hours. The temperature was then increased to 60°C and reaction was carried out for 4 hours. Then the enzyme preparation was deactivated under 65°C for 1 hour. After that, the solution was subjected to steps of centrifugal separation using a centrifugal apparatus, sterilization, concentration in a concentration device, and spray drying using a spray-drying tower to form the product. Type 2700 biochemistry analyzer was used to detect the glutamic acid content of the resulted product. Results showed that glutamic acid content was 10.8% in final product.

### Example 2

The same procedure as example 1, except that the addition of protease was 2‰, the addition of glutamine transaminase was 1‰, with pH value of 6.0-6.5 and temperature of 55°C.

### Example 3

The same procedure as example 1, except that the addition of protease was 1‰, the addition of glutamine transaminase was 2‰, with pH value of 6.0-6.5 and temperature of 55°C.

### Control example 1

Same methods and procedures as in examples were employed to perform control example, except that papain alone was used in enzymolysis step, with the absence of glutamine transaminase. Glutamic acid content turned to be 4.6% in the resulted product, showing that the use of glutamine transaminase in the present invention greatly enhanced the glutamic acid content in the product.

### Control example 2

Same methods and procedures as in example I were employed to perform control example, except that glutamine transaminase alone was used in enzymolysis step, with the absence of papain. Glutamic acid content turned to be 3.0% in the resulted product.

**Table 2 Glutamic acid content in yeast extract**

| | Example 1 | Example 2 | Example 3 | Control example 1 | Control example 2 |
|---|---|---|---|---|---|
| Protease addition ‰ | 1.5 | 2 | 1 | 3 | 0 |
| Glutamine transaminase addition ‰ | 1.5 | 1 | 2 | 0 | 3 |
| pH of enzymolysis | 6.0-6.5 | 6.0-6.5 | 6.0-6.5 | 6.0-6.5 | 6.0-6.5 |
| Temperature of enzymolysis °C | 55 | 55 | 55 | 55 | 55 |
| Glutamic acid content in extract % | 10.8 | 10.2 | 11.2 | 4.6 | 3.0 |

The above experimental data showed that by cultivating specific yeast strain and adding protease and glutamine transaminase in a certain ratio into resulted protein-abundant culture, glutamic acid content of yeast extract in the present invention is 10% or more, which is higher than that in the prior yeast extract product. Besides, the producing method was easy to carry out and suitable for commercial production on large scale.

The above-mentioned examples are preferred ones only, and are not used to limit the present invention. Those skilled in the art should understand that various modifications and transformations could be taken on the present invention.

## Claims

1. A method for producing the yeast extract, wherein said yeast extract is produced with a yeast strain culture as raw material, wherein the deposit accession number of said yeast strain is CCTCC NO: M205130 and the protein content of said culture is higher than 60%, said method comprising:
A) cultivating yeast strain whose deposit accession number is CCTCC NO: M205130 in culture media containing nitrogen sources to obtain culture and preparing said culture into yeast cream, wherein aqua ammonia as the nitrogen source in culture media accounts for 2% or more of the total mass of culture media;
B) adjusting the pH value of said yeast cream to pH 4.5-6.8 and heating it to 45°C-60°C;
C) adding protease and glutamine transaminase into said yeast cream to perform autolytic enzymolysis reaction, wherein said protease is papain and is added in an amount of 1.5‰ relative to the total weight of yeast in said yeast cream;
D) increasing the temperature to 60°C and then performing centrifugation; and
E) obtaining said yeast extracts after sterilization, concentration and spray drying.

2. The producing method according to claim 1, wherein the mass ratio of said protease and glutamine transaminase is 1:1.

3. The producing method according to claim 1, wherein said glutamine transaminase is added in an amount of 1.5‰ relative to the total weight of yeast in said yeast cream.

4. The producing method according to claim 1, wherein said autolytic enzymolysis reaction is performed for about 16-20 hours.

5. The producing method according to claim 1, wherein the pH value of autolytic enzymolysis reaction is in the range of 4.5-6.8.

## Patentansprüche

1. Verfahren zum Herstellen des Hefeextrakts, worin der Hefeextrakt hergestellt wird mit einer Hefestammkultur als Rohmaterial, worin die Hinterlegungsnummer des Hefestamms CCTCC NO: M205130 ist und der Proteingehalt der Kultur höher als 60 % ist, wobei das Verfahren umfasst:
A) Kultivieren eines Hefestamms, dessen Hinterlegungsnummer CCTCC NO: M205130 ist in Kulturmedium, das Stickstoffquellen enthält, um eine Kultur zu erhalten, und Verarbeiten der Kultur in eine Hefecreme, worin wässriger Ammoniak in dem Kulturmedium als die Stickstoffquelle zu 2 % oder mehr der Gesamtmasse des Kulturmediums beiträgt;
B) Einstellen des pH-Werts der Hefecreme auf pH-Wert 4,5-6,8 und Erwärmen auf 45 °C-60 °C;
C) Zugeben von Protease und Glutamintransaminase in die Hefecreme, um autolytische Enzymolysereaktion durchzuführen, worin die Protease Papain ist und in einer Menge von 1,5 ‰ relativ zum Gesamtgewicht der Hefe in der Hefecreme zugegeben wird;
D) Erhöhen der Temperatur auf 60 °C und dann Durchführen einer Zentrifugation; und
E) Erhalten des Hefeextrakts nach Sterilisation, Konzentrieren und Sprühtrocknen.

2. Herstellungsverfahren nach Anspruch 1, worin das Masseverhältnis der Protease und Glutamintransaminase 1:1 ist.

3. Herstellungsverfahren nach Anspruch 1, worin die Glutamintransaminase in einer Menge von 1,5 ‰ relativ zum Gesamtgewicht der Hefe in der Hefecreme zugegeben wird.

4. Herstellungsverfahren nach Anspruch 1, worin die autolytische Enzymolysereaktion für etwa 16-20 Stunden durchgeführt wird.

5. Herstellungsverfahren nach Anspruch 1, worin der pH-Wert der autolytischen Enzymolysereaktion im Bereich von 4,5-6,8 ist.

## Revendications

1. Procédé de production de l'extrait de levure, dans lequel ledit extrait de levure est produit avec une culture de souche de levure en tant que matière première, où le numéro d'accession du dépôt de ladite souche de levure est CCTCC NO : M205130 et la teneur en protéines de ladite culture est supérieure à 60 %, ledit procédé comprenant :
A) la culture de la souche de levure dont le numéro d'accession du dépôt est CCTCC NO : M205130 dans un milieu de culture contenant des sources d'azote pour obtenir la culture et la préparation de ladite culture dans de la crème de levure, où l'ammoniac aqueux en tant que source d'azote dans le milieu de culture représente 2 % ou plus de la masse totale du milieu de culture ;
B) l'ajustement de la valeur du pH de ladite crème de levure à pH 4,5 à 6,8 et son chauffage à 45 °C à 60 °C ;
C) l'addition d'une protéase et d'une glutamine transaminase dans ladite crème de levure pour effectuer une réaction d'enzymolyse autolytique, où ladite protéase est la papaïne et est ajoutée dans une quantité de 1,5 ‰ par rapport au poids total de la levure dans ladite crème de levure ;
D) l'augmentation de la température à 60 °C et ensuite la réalisation d'une centrifugation ; et
E) l'obtention desdits extraits de levure après stérilisation, concentration et séchage par pulvérisation.

2. Procédé de production selon la revendication 1, dans lequel le rapport massique desdites protéase et glutamine transaminase est de 1/1.

3. Procédé de production selon la revendication 1, dans lequel ladite glutamine transaminase est ajoutée dans une quantité de 1,5 ‰ par rapport au poids total de la levure dans ladite crème de levure.

4. Procédé de production selon la revendication 1, dans lequel ladite réaction d'enzymolyse autolytique est effectuée pendant environ 16 à 20 heures.

5. Procédé de production selon la revendication 1, dans lequel la valeur du pH de la réaction d'enzymolyse autolytique est située dans la plage de 4,5 à 6,8.
